# FASCICULE DE BREVET EUROPEEN

(11) **EP 1 165 108 B1**
(45) Date de publication et mention de la délivrance du brevet: **28.05.2003**
(21) Numéro de dépôt: 00915254.7
(22) Date de dépôt: 30.03.2000
(51) Int. Cl.: A61K 35/78, A61P 35/00

(54) **UTILISATION D'UN EXTRAIT DE SERENOA REPENS POUR LA FABRICATION D'UN MEDICAMENT DESTINE AU TRAITEMENT DU CANCER DE LA PROSTATE**
VERWENDUNG EINES SERENOA REPENS EXTRAKTES ZUR HERSTELLUNG EINES ARZNEIMITTELS ZUR BEHANDLUNG VON KREBSERKRANKUNGEN DER PROSTATA
USE OF A SERENOA REPENS EXTRACT FOR THE PRODUCTION OF A MEDICAMENT TO TREAT PROSTATE CANCER

(30) Priorité: 30.03.1999 FR 9903959
(43) Date de publication de la demande: 02.01.2002
(73) Titulaire: PIERRE FABRE MEDICAMENT, 92100 Boulogne (FR)
(72) Inventeur: FABRE, Pierre, F-81106 Castres (FR); RAYNAUD, Jean-Pierre, F-75016 Paris (FR); COUSSE, Henri, F-31860 Pins Justaret (FR)
(74) Mandataire: Ahner, Francis
(86) Numéro de dépôt international: FR0000804
(87) Numéro de publication internationale: WO00057892

(56) Documents cités:
- WO-A-93/20832
- FR-A- 2 465 486
- FR-A- 2 480 754
- US-A- 5 665 393
- LOUIS KURITZKY: "BENIGN PROSTATIC HYPERPLASIA" COMPREHENSIVE THERAPY, vol. 24, no. 3, mars 1998 (1998-03), pages 130-135, XP000863205 AMERICAN SOCIETY OF CONTEMPORARY MEDICINE AND SURGERY, CHICAGO, IL, US ISSN: 0098-8243

## Description

La présente invention concerne le domaine du traitement du cancer de la prostate qui, à ce jour, repose sur plusieurs voies thérapeutiques dépendantes du degré d'évolution de la maladie. Le traitement hormonal du cancer métastatique de la prostate ayant franchi la capsule, s'appuie désormais principalement sur plusieurs catégories de médicaments agissant à des niveaux différents de l'axe hypothalamo-gonadique.

L'efficacité et les limites des traitements hormonaux sont à présent plus ou moins définis. Ces limites sont fixées à la fois par les effets secondaires, en particulier vasculaires pour les oestrogènes à fortes doses et sexuels, gastriques et pulmonaires pour les antiandrogènes, et par l'émergence de résistances d'emblée ou secondaires.

L'attitude actuelle paraît se focaliser, au moins à la phase initiale du traitement, autour du blocage androgénique complet (inhibition de la sécrétion testiculaire d'androgènes et inhibition de l'activité des androgènes résiduels sur l'organe cible). En effet, l'absence de certitude absolue sur l'intérêt de poursuivre cette association au long cours fait parfois préférer un blocage androgénique complet au moment de la mise en route d'un traitement par un agoniste de la LHRH, afin de prévenir les flambées initiales avec poursuite ultérieure de l'agoniste seul.

Le pronostic des cancers de la prostate évolués et l'androgénodépendance d'une grande majorité d'entre eux, incite à éradiquer le plus complètement possible l'ambiance androgénique, y compris surrénalienne. La supériorité d'un traitement combiné au long cours (castration chirurgicale ou agoniste de la LHRH associé à une thérapeutique anti-androgène) sur la durée de vie est de plus en plus confirmée.

Quel que soit le traitement hormonal initial appliqué, la réponse dépasse rarement deux ans jusqu'à l'installation de la phase d'hormonorésistance. Ni le changement d'hormonothérapie, ni le recours à la chimiothérapie, n'ont jusqu'à présent permis de prolonger de façon significative la médiane de survie qui reste de l'ordre de dix huit mois.

L'hormonothérapie, dans le but de supprimer les hormones responsables de la croissance tumorale, implique généralement l'administration d'agonistes LHRH seuls ou associés à des agents anti-androgènes (voir par exemple brevet FR 2 465 486).

Les cellules cancéreuses de la prostate peuvent également être partiellement éliminées par radiothérapie et/ou par des interventions chirurgicales.

Pour traiter le cancer de la prostate, le praticien dispose donc à ce jour de plusieurs moyens d'intervention. On mentionnera tout d'abord la chirurgie, puis les analogues de la LHRH, et enfin les anti-androgènes utilisés soit en association à la castration médicale ou chirurgicale soit en monothérapie, et enfin la radiothérapie.

Cependant aucun de ces moyens n'est jugé parfaitement satisfaisant à ce jour. Si l'on sait que l'hormonosensibilité justifie l'usage de l'honnonothérapie, les études conduites à ce jour ne permettent pas de définir de façon précise les modalités optimales du rapport bénéfice/risque.

En effet, la castration chimique entraîne l'impuissance et la baisse de la libido, cet effet pouvant être réversible à l'arrêt du traitement mais avec risque de récidive à plus ou moins long terme.

La présente invention constitue une amélioration déterminante dans le traitement du cancer de la prostate. Elle vise plus particulièrement l'utilisation d'un extrait lipido-stérolique de Serenoa Repens pour la fabrication d'un médicament destiné à être administré isolément ou en association, de manière simultanée, séparée ou étalée dans le temps, avec une prostatectomie, une radiothérapie, et/ou une hormonothérapie, en vue du traitement ou de la prévention du cancer de la prostate.

Dans le cadre de la présente invention, il a été observé que l'extrait lipido-stérolique de Serenoa Repens, jouait le rôle d'un inducteur d'apoptose, permettant le traitement en première intention avant chirurgie ou radiothérapie, afin d'éviter la dissémination de la tumeur à l'extérieur de la capsule prostatique.

En outre, l'extrait lipido-sterolique de Serenoa Repens associé à un traitement hormonal, permet à l'arrêt du traitement hormonal de contrôler la progression tumorale par l'induction de la mort cellulaire. Pareil traitement séquentiel permet d'améliorer considérablement le rapport bénéfice/risque.

Enfin, après la prostatectomie et/ou après la radiothérapie, l'extrait lipido-stérolique de Serenoa Repens retarde la progression des cellules cancéreuses qui auraient échappé au traitement.

Pour illustrer le traitemer.t hormonothérapique associé à l'administration de l'extrait lipido-stérolique de Serenoa Repens selon l'invention, on mentionnera tout d'abord les agonistes ou antagonistes de la LHRH, en particulier la triptoréline, la leuproréline, la nafaréline, la goséréline, la buséréline, ainsi que les agents anti-androgènes non stéroïdiens tels que le flutamide, le nilutamide ou encore le bicalutamide.

Selon une variante avantageuse de la présente invention, ledit médicament est administré en association avec un antiandrogène associé à un agoniste ou à un antagoniste de LHRH.

L'extrait lipide-stérolique de Serenoa Repens, a jusqu'à présent été utilisé pour traiter l'hyperplasie bénigne de la prostate. Dans le cadre de la présente invention, il a été constaté de façon inattendue que l'extrait lipido-stérolique de Serenoa Repens pouvait en fait jouer le rôle d'un inducteur d'apoptose des cellules stromales et épithéliales prostatiques.

A la suite de ces observations plusieurs essais cliniques ont été conduits, qui ont permis de situer l'intérêt de l'extrait lipido-stérolique de Serenoa Repens dans l'arsenal thérapeutique utilisé pour le traitement du cancer de la prostate.

Il convient de rappeler que l'extrait lipido-stérolique de Serenoa Repens est un extrait titré obtenu à partir de Serenoa Repens (Sabal Serrulata, Saw Palmeto).

Cet extrait est plus particulièrement obtenu à l'aide de solvants hydrophobes tels que CO2 supercritique ou d'hexane. Un tel extrait ne contient pas de phyto-estrogènes, ce qui le différencie des isoflavones de soja ou de tout autre phyto-estrogènes comme décrits par exemple dans New England J. of Medecine Vol 339 (12) p. 785-791.

Cet extrait peut être titré en acides gras libres (acides laurique + oléique = 65 %) et en alcools gras traceurs de la partie insaponifiable (0,2 %). Pour une description plus complète du procédé de fabrication d'un tel extrait, on pourra par exemple se reporter à la description du brevet FR 2 480 754.

A titre d'exemples non limitatifs, l'invention sera illustrée par les trois essais cliniques positifs dont les protocoles sont énoncés ci-après.

### 1ère étude :

L'extrait lipido-stérolique de Serenoa Repens a été utilisé à la dose de 320 mg administré trois fois par jour.

Les patients sont des hommes âgés de 50 à 75 ans, avec un cancer de la prostate localisé (stade T1C, T2A et T2B) détecté par biopsie positive.

Cette étude a montré dans les groupes traités par extrait lipido-stérolique Serenoa Repens une augmentation significative de la mort cellulaire par rapport à un groupe non traité (en attente de chirurgie).

Par rapport aux patients traités par LHRH et anti-androgène pendant cette même période de trois mois, l'effet sur l'apoptose a été similaire (augmentation significative de l'indice apoptotique), mais dans les groupes traités par extrait lipido-stérolique de Serenoa Repens la qualité de vie a été maintenue plus particulièrement en ce qui concerne la sexualité (évaluée par un questionnaire approprié et validé).

### 2ème étude :

L'extrait lipido-stérolique de Serenoa Repens a été administré à la dose journalière de 960 mg pendant six mois à des patients à l'arrêt d'un traitement hormonal de trois mois.

Un groupe témoin n'a pas reçu de traitement de substitution, au bout de six mois, une différence significative a été obtenue sur le taux d'échappement biologique, ce qui démontre un intérêt de l'extrait lipido-stérolique de Serenoa Repens pour un traitement séquentiel.

Compte-tenu de ces résultats, une étude au long cours a été entreprise sur cinq ans, dont le protocole est indiqué ci-dessous.

### 3ème étude :

Après radiothérapie ou prostatectomie radicale, chez certains patients, du fait de la difficulté d'intervention chirurgicale lié à l'accès difficile, un traitement complémentaire est nécessaire pour contrôler l'évolution des cellules non éliminées. En général après chirurgie, une période d'attente sans traitement est observée, pendant laquelle l'innocuité de l'extrait lipido-stérolique de Serenoa Repens a été mise à profit pour être administré pendant cinq ans à la dose de 640 mg/jour.

Les résultats de cette étude sont comparés en terme de bénéfice (risque d'échappement et récidive) aux patients n'ayant reçu aucun traitement.

Tous les ans un contrôle est effectué, en cas de nécessité un traitement hormonal est substitué.

Ces études cliniques illustrent le positionnement thérapeutique de l'extrait lipido-stérolique de Serenoa Repens en tant qu'inducteur d'apoptose dans les traitements néo-adjuvants.

On précisera enfin que l'extrait lipido-stérolique de Serenoa Repens peut être utilisé dans le cadre de la présente invention sous diverses formes de préparations pharmaceutiques. Il peut en particulier s'agir de gélules ou de capsules.

L'extrait lipido-stérolique de Serenoa Repens constitue par conséquent un inducteur d'apoptose utilisable soit en première intention pour la prévention ou pour le traitement d'un cancer débutant, soit en traitements séquentiels d'hormonothérapie.

L'invention s'étend également à l'utilisation de l'extrait lipido-stérolique de Serenoa Repens pour la fabrication d'un médicament destiné à être administré dans le cadre d'un traitement associé, en particulier à l'hormonothérapie. Les études cliniques ont objectivé la bonne tolérance et l'efficacité de cet inducteur utilisable seul, mais dont les effets complémentaires aux traitements hormonaux améliorent nettement la prise en charge thérapeutique.

L'extrait lipido-stérolique de Serenoa Repens constitue donc un néo-adjuvant utilisable dans le traitement du cancer de la prostate à tous les stades de développement de la maladie, seul ou associé aux autres traitements existants et plus particulièrement aux traitements impliquant des agonistes ou antagonistes de la LHRH.

## Revendications

1. Utilisation d'un extrait lipido-stérolique de Serenoa Repens pour la fabrication d'un médicament destiné à être administré isolément ou en association, de manière simultanée, séparée ou étalée dans le temps, avec une prostatectomie, une radiothérapie, et/ou une hormonothérapie, en vue de la prévention et/ou du traitement du cancer de la prostate.

2. Utilisation selon la revendication 1, **caractérisée en ce que** ledit médicament est destiné à être administré en association avec un agoniste ou antagoniste de la LHRH, en particulier la triptoréline, la leuproréline, la nafaréline, la goséréline, et la buséréline.

3. Utilisation selon l'une des revendications 1 ou 2, **caractérisée en ce que** le médicament est administré en association avec un antiandrogène non stéroïdien, en particulier, le flutamide, le nilutamide et/ou le bicalutamide.

4. Utilisation selon l'une des revendications 1, 2 ou 3, **caractérisée en ce que** ledit médicament est administré en association avec un antiandrogène associé à un agoniste ou à un antagoniste de LHRH.

## Patentansprüche

1. Verwendung eines lipido-sterolischen Extrakts von Serenoa Repens für die Herstellung eines Medikaments, das dazu bestimmt ist, allein oder auf gleichzeitige, getrennte oder zeitlich gestaffelte Weise in Verbindung mit einer Prostatektomie, einer Radiotherapie und/oder einer Hormontherapie im Hinblick auf die Verhütung und/oder die Behandlung von Prostatakrebs verabreicht zu werden.

2. Verwendung nach Anspruch 1, **dadurch gekennzeichnet, dass** das Medikament dazu bestimmt ist, in Verbindung mit einem Agonisten oder Antagonisten von LHRH, insbesondere Triptorelin, Leuprorelin, Nafarelin, Goserelin und Buserelin, verabreicht zu werden.

3. Verwendung nach einem der Ansprüche 1 oder 2, **dadurch gekennzeichnet, dass** das Medikament in Verbindung mit einem nicht-steroidalen Antiandrogen, insbesondere Flutamid, Nilutamid und/oder Bicalutamid, verabreicht wird.

4. Verwendung nach einem der Ansprüche 1, 2 oder 3, **dadurch gekennzeichnet, dass** das Medikament in Verbindung mit einem Antiandrogen verabreicht wird, das mit einem Agonisten oder einem Antagonisten vorn LHRH assoziiert ist.

## Claims

1. Use of a lipid-sterol extract of Serenoa Repens to manufacture a medicinal product to be administered individually or in combination, simultaneously, separately or sequentially over time, with a prostatectomy, radiotherapy and/or a hormone therapy, for the prevention and/or treatment of prostate cancer.

2. Use according to Claim 1, **characterized in that** said medicinal product is to be administered in combination with an LHRH agonist or antagonist, in particular triptorelin, leuprorelin, nafarelin, goserelin or buserelin.

3. Use according to either of Claims 1 and 2, **characterized in that** the medicinal product is administered in combination with a nonsteroidal antiandrogen, in particular flutamide, nilutamide and/or bicalutamide.

4. Use according to one of Claims 1, 2 and 3, **characterized in that** said medicinal product is administered in combination with an antiandrogen combined with an LHRH agonist or antagonist.
